# EUROPEAN PATENT APPLICATION

(11) **EP 4 169 520 A1**
(43) Date of publication of application: **26.04.2023**
(21) Application number: 21829916.2
(22) Date of filing: 22.06.2021
(51) Int. Cl.: A61K 35/74, A61K 39/395, A61K 45/06, A61P 35/00, C07K 16/28, A61K 39/00

(54) **METHOD AND COMPOSITION FOR ENHANCING CANCER TREATMENT EFFICACY OF BACTERIA EXTRACELLULAR VESICLES**

(30) Priority: 22.06.2020 KR 20200076053
(71) Applicant: Rosetta Exosome Co., Ltd., Seongnam-si, Gyeonggi-do 13494 (KR)
(72) Inventor: GHO, Yong Song, Pohang-si, Gyeongsangbuk-do 37673 (KR); LEE, Jaemin, Pohang-si, Gyeongsangbuk-do 37673 (KR); GO, Gyeongyun, Pohang-si, Gyeongsangbuk-do 37673 (KR); LEE, Jaewook, Pohang-si, Gyeongsangbuk-do 37673 (KR); BAE, Seoyoon, Pohang-si, Gyeongsangbuk-do 37673 (KR)
(74) Representative: Berggren Oy
(86) International application number: PCT/KR2021/007834
(87) International publication number: WO 2021/261891

(57) **Abstract**

The present invention relates to a method and composition for enhancing the cancer treatment efficacy of bacteria extracellular vesicles, and more particularly, to a method and composition for enhancing the cancer treatment efficacy of bacteria extracellular vesicles by using a single immune checkpoint inhibitor or various combinations of two or more types of immune checkpoint inhibitors. This composition exhibits a synergic effect compared to single dosage forms of the respective inhibitors and exhibits an action of alleviating adverse effects caused by high doses of the single dosage forms, and thus may be usefully utilized in the development of cancer drugs.

## Description

### TECHNICAL FIELD

This application claims the priority of Korean Patent Application No. 10-2020-0076053, filed on June 22, 2020, the entirety of which is a reference of the present application.

The present invention relates to a method and composition for enhancing the cancer treatment effect of bacterial extracellular vesicles, and more particularly, to a method and composition for enhancing the cancer treatment effect of bacterial extracellular vesicles using a single immune checkpoint inhibitor or various combinations of two or more types of immune checkpoint inhibitors.

### BACKGROUND ART

Cancer is one of the major causes of deaths, but is curable in some cases if detected in the early stages. However, it is difficult to treat cancers if the cancer is advanced to a certain extent, particularly in the cases involving metastases. Recently, it has been known and recognized that cancers are not regulated by the immune systems, due to immunosuppressive environments present in most cancers. As a result, various methods have been developed to promote the immune systems to regulate cancers. Cancer immunotherapy is a strategy to treat cancers by using a body's immune functions, and has received much interest and expectation since William B. Coley observed tumor regression by administrating bacterial mixtures known as Coley's toxin in the late 19th century. It was urgent to develop consistent and reproducible cancer immunotherapies, because various cancer immunotherapies, attempted in the 1980s and 1990s, resulted in disappointing outcomes. Recently, cancer immunotherapies became realized as monoclonal antibodies against immune checkpoint inhibitorss showed remarkable therapeutic effects on various cancers. By blocking one or more immune checkpoints, immune checkpoint inhibitors remove one of the barriers in the immune systems, and inhibit the immunosuppressive tumor microenvironment. Immune checkpoint inhibitors include monoclonal antibodies against cytotoxic T-lymphocyte-associated protein 4 (CTLA4), programmed cell death protein 1 (PD1) and programmed death-ligand 1 (PDL 1).

The immune response of antigen-specific T lymphocytes is very complex and elaborately regulated, and it is a representative specific immune response, such as killing antigen-containing cells, including tumor cells, by cytotoxic T lymphocytes. Along with antigen recognition, co-stimulatory signals are required to activate antigen-specific T lymphocytes, and these signals are achieved by simultaneous binding of CD80 and CD40 expressed on antigen-presenting cells to their corresponding ligands, such as CD28 and CD40L, expressed on the surface of T lymphocytes.

However, activated T lymphocytes are inactivated through the activation of co-inhibitory signals after some time, thereby preventing tissue damages caused by excessive immune stimulation. There are various co-inhibitory signals: for examples, 1) CTLA4 of T lymphocytes binds to its ligands, CD80 and CD86, on antigen-presenting cells, and inactivates naive or memory T lymphocytes. 2) PD1 of T lymphocytes binds to its ligands, PDL1 and PD L2, on antigen-presenting cells, and inhibits T lymphocyte functions in the peripheral tissues. The immune function of the human body regulates overall T lymphocyte functions by adjusting these co-stimulatory and co-inhibitory signals simultaneously with recognizing antigens. These signals are called immune checkpoints.

The immune function of the human body eradicate tumor cells by detecting tumor-specific neo-antigens expressed in tumor cells. On the contrary, tumor cells activate inhibitory immune checkpoints by altering tumor microenvironments to evade these immune attacks, or avoid the attacks of tumor-specific killer T lymphocytes through T lymphocyte immune tolerance or immuno-editing. Recently, it has been found that there are therapeutic effects on various cancers, such as malignant melanoma, renal cancers, or non-small cell lung cancers, by activating tumor-specific cytotoxic T lymphocytes, which were inhibited, using immune checkpoint inhibitors (monoclonal antibodies against CTLA4, PD1, or PDL1). However, the immune checkpoint inhibitors were effective only in some cancers, and only in some patients with these cancers, suggesting that different types of immunosuppressive mechanisms act in various cancers.

Despite dramatic cancer therapeutic effects of the immune checkpoint inhibitors, there are differences in therapeutic effects depending on patients. Thus, many studies are actively conducted on the specificities of tumor microenvironments, such as expression levels of CTLA4, PD1, PDL1, or PDL2, as well as the number of T lymphocytes, in cancer patients, and various combination therapies. Recently, it has been found that cancer therapeutic effects of immune checkpoint inhibitors varied depending on the distribution of intestinal bacteria in the patients, and that specific intestinal bacteria could increase the cancer therapeutic effects of immune checkpoint inhibitors in animal experiments. However, it has not been found how these specific intestinal bacteria and which materials derived from intestinal bacteria enhanced the cancer therapeutic effects of immune checkpoint inhibitors

Meanwhile, it has been reported that all cells, including Gram-negative and Gram-positive bacteria, naturally release extracellular vesicles. The extracellular vesicles derived from Gram-negative bacteria are also known as outer membrane vesicles. Bacterial extracellular vesicles have diameters ranging from 20 to 200 nm, and have various biologically active materials, such as proteins, lipids, genetic materials (DNA and RNA) and peptidoglycans. The extracellular vesicles released from Gram-negative and Gram-positive bacteria also have virulence factors, such as lipopolysaccharide (LPS) and lipoteichoic acid (LTA), respectively. The bacterial extracellular vesicles function as communicators by intra-specifically delivering proteins or genetic materials and cell signaling. They also contribute to killing competing organisms and enhancing bacterial survival. In addition, they deliver toxins to the host cells to regulate the pathogenesis of bacterial infectious diseases.

Recently, it has been shown that various bacterial extracellular vesicles have direct therapeutic effects on various diseases including cancers, and they can be utilized to drug carriers for treating these diseases. They have been clinically used or developed as vaccine carriers to prevent or treat various diseases such as meningitis.

In addition, bacterial extracellular vesicles have various components that can activate the immune systems. As the bacterial extracellular vesicles are free of living bacteria, they are safer than bacteria themselves. After intravenously administering bacterial extracellular vesicles to tumor-bearing mice, it has been reported that the bacterial extracellular vesicles induce long-term anti-tumor immune responses that effectively eliminate the tumor tissues without prominent adverse effects (Nature Communications. 8:626, 2017). Furthermore, as the bacterial extracellular vesicles are nano-sized entities, they are specifically accumulated in the tumor tissues by enhanced permeability and retention (EPR) effects. They are assumed to induce anti-tumor immune responses by inducing the release of IFN-γ from natural killer cells and T lymphocytes (Nature Communications. 8:626, 2017).

However, it has not been elucidated whether the combination of bacterial extracellular vesicles and anti-cancer agents with different mechanisms of action, including immune checkpoint inhibitors, could enhance therapeutic effects against cancers.

### DISCLOSURE

### TECHNICAL PROBLEM

The present inventors tried to develop methods for enhancing cancer therapeutic effects and combinatorial drugs for treating cancers, which effectively inhibit tumor growth and do not exhibit adverse effects. The inventors found that the purposes could be achieved by administering bacterial extracellular vesicles in combination with immune checkpoint inhibitors, and then completed the present invention.

### TECHNICAL SOLUTION

To achieve the object of the present invention, the present invention provides a method for enhancing cancer therapeutic effects of bacterial extracellular vesicles by administering the bacterial extracellular vesicles in combination with immune checkpoint inhibitors, and a pharmaceutical composition for preventing or treating cancer including a bacterial extracellular vesicle and an immune checkpoint inhibitor as active ingredients.

In addition, it provides a pharmaceutical composition for preventing or treating cancer consisting of a bacterial extracellular vesicle and an immune checkpoint inhibitor.

In addition, it provides a pharmaceutical composition for preventing or treating cancer essentially consisting of a bacterial extracellular vesicle and an immune checkpoint inhibitor.

Typically, Gram-negative bacteria have two membranes consist of the outer membrane and the inner membrane (or cytoplasmic membrane), whereas Gram-positive bacteria only have a cell membrane (cytoplasmic membrane). In the present invention, the bacterial extracellular vesicle collectively refers to a membrane vesicle naturally released (native extracellular vesicle) from bacteria (including transformed bacteria), and a membrane vesicle which is artificially produced (artificial extracellular vesicle). The native extracellular vesicle is called as an extracellular vesicle, an outer membrane vesicle, a shedding vesicle, a microvesicle, a microparticle, or an exosome, and it can be understood that all these entities are included in the extracellular vesicles of the present invention.

The artificial extracellular vesicle is a membrane vesicle artificially formed by a method selected from the group consisting of extrusion, sonication, cell lysis, pH change, temperature change, homogenization, freezing-thawing, electroporation, mechanical disruption, and treatment with various chemical materials such as antibiotics or surfactants (detergents) or a combination thereof with respect to bacteria (or including transformed bacteria). In addition, it can be understood that all of these entities are included in the extracellular vesicles of the present invention, and methods of forming the artificial extracellular vesicles are not limited thereto. In addition, in the case of Gram-negative bacteria, the artificial extracellular vesicles include outer membrane-derived vesicles (OMDVs), inner membrane-derived vesicles (IMDVs), and inner-outer membrane-derived extracellular vesicles (e.g., ones with both the outer and inner membrane components, ones with the inner membrane present inside the outer membrane, ones with the outer membrane present inside the inner membrane, etc.), and cell membrane-derived vesicles (CMDVs) including the OMDVs, the IMDVs, and the inner-outer membrane-derived vesicles, and have diameters ranging from 20 nm to 1,000 nm. In addition, it can be understood that all these entities are included in the artificial extracellular vesicles in the present invention.

In the present invention, the types of bacteria are not limited. Specifically, the bacteria may be Gram-negative bacteria or Gram-positive bacteria.

Example of the Gram-negative bacteria may include *Escherichia* genus, *Helicobacter* genus, *Hemophilus* genus, *Neisseria* genus, *Cyanobacterium* genus, *Klebsiella* genus, *Acetobacter* genus, *Acinetobacter* genus, *Enterobacter* genus, *Chlamydia* genus, *Vibrio* genus, *Pseudomonas* genus, *Salmonella* genus, *Thiobacter* genus, *Borrelia* genus, *Burkholderia* genus, *Serratia* genus, *Treponema* genus, *Rikenella* genus, *Alistipes* genus, *Marinilabilia* genus, *Proteus* genus, *Enhydrobacter* genus, *Methylobacterium* genus, *Morganella* genus, *Cupriavidus* genus, *Yersinia* genus, *Shigella* genus, *Legionella* genus, *Stenotrophomonas* genus, and *Moraxella* genus bacteria. Examples of the Gram-positive bacteria may include *Bacillus* genus, *Nocardia* genus, *Clostridium* genus, *Propionibacterium* genus, *Actinomyces* genus, *Enterococcus* genus, *Corynebacterium* genus, *Listeria* genus, *Lactobacillus* genus, *Gardnerella* genus, *Mycobacterium* genus, *Mycoplasma* genus, *Staphylococcus* genus, *Streptomyces* genus, *Micrococcus* genus, *Streptococcus* genus, *Bifidobacterium* genus, *Anaerostipes* genus, *Coprococcus* genus, *Atopobium* genus, *Faecalibacterium* genus, *Lactococcus* genus, and *Bacteroides* genus bacteria and the like.

In an aspect of the present invention, the bacteria may be transformed bacteria. The transformed bacteria include bacteria transformed to attenuate the toxicity of the extracellular vesicles, and examples thereof may include bacteria in which an endotoxin production gene is deleted or modified. Preferably, the transformed bacteria may be bacteria transformed so that *msbB* gene is deleted (*ΔmsbB*), more preferably Gram-negative bacteria transformed so that the *msbB* gene is deleted, most preferably *Escherichia coli* transformed so that the *msbB* gene is deleted, but are not limited thereto.

In addition, the bacteria include bacteria transformed to release extracellular vesicles well.

In addition, the bacteria include bacteria transformed to target a specific cell or tissues, and examples thereof may include bacteria transformed to target tumor blood vessels, tumor tissues, or tumor cells. In addition, the bacteria used in the present invention include bacteria transformed to be fused with the cell membrane of target cells, bacteria transformed to express materials for treating and/or diagnosing diseases, and bacteria transformed so that the specific material is inhibited and another specific material is expressed simultaneously, but are not limited thereto.

Transformation of bacterial cells can include a method of increasing or changing the expression of materials such as proteins by stimulating cells, and a method of increasing or inhibiting expression of proteins through gene introduction, but is not limited thereto.

The method for increasing the expression of the specific protein may use plasmid DNA, RNA, or phage, and may use all generally known methods such as heat shock, calcium phosphate precipitation, lipofectamine transfection, electroporation, and microinjection. In order to inhibit the expression of the specific protein, a specific gene can be removed from cells, and all generally known methods such as a method using antisense RNA may be used.

In an aspect of the present invention, it is characterized that the bacteria may be bacteria cultured in a chemically defined medium.

In the present invention, the "chemically defined medium" is different from a "natural medium" using naturally derived materials with unclear compositions such as serum and tissue extracts, and refers to a synthetic medium prepared only with materials with clear compositions and chemical properties. It may be preferred that the bacteria are cultured in a chemically defined medium to produce extracellular vesicles that exhibit a uniform effect.

In the present invention, the chemically defined medium may be selected from the group consisting of M9 medium, Dulbecco's modified Eagle's medium (DMEM medium), and Roswell Park Memorial Institute medium 1640 (RPMI 1640 medium), but is not limited thereto.

In an aspect of the present invention, the bacteria may be transformed to express one or more selected from the group consisting of immune checkpoint proteins, cell adhesion molecules, various antibodies including immune checkpoint inhibitors, proteins (cytokine, chemokine, growth factor) which enhance therapeutic effects such as IL-12 or interferon gamma, targeting proteins, cell membrane fusion materials, and fusion proteins thereof, but are not limited thereto. These materials may be effectively displayed on the surface of the extracellular vesicles by using a fuson protein of various proteins such as an outer membrane protein or an inner membrane protein (e.g., a fusion protein of PrsA (an inner membrane protein) and human epidermal growth factor (EGF; a targeting protein)).

Preferably, the bacteria may be bacteria transformed to display human immune checkpoint proteins (PD1, PDL1, etc.) or antibodies thereto to inhibit the effects of immune checkpoint inhibitors on the surface of the extracellular vesicles, more preferably Gram-negative bacteria transformed to express human immune checkpoint proteins (PD1, PDL1, etc.) or antibodies thereto, most preferably *E. coli* transformed to express human immune checkpoint proteins (PD1, PDL1, etc.) or antibodies thereto, but are not limited thereto.

In an exemplary embodiment of the present invention, *E. coli ΔmsbB*, in which the toxicity of lipopolysaccharides is attenuated, is transformed with pHCE-prsA-EGF vector expressing a fusion protein of human EGF and a bacterial inner membrane protein PrsA to produce *E. coli ΔmsbB*-prsA-EGF, and the extracellular vesicles were isolated therefrom.

In an aspect of the present invention, the pharmaceutical composition may further include a drug that inhibits the toxicity by bacterial extracellular vesicles, immune checkpoint inhibitors, and combinations thereof. The drug includes a drug that inhibit the toxicity caused by endotoxins (e.g., polymyxin B), an anti-inflammatory agent including dexamethasone as well as aspirin, an anticoagulant, and a cyclooxygenase (COX) inhibitor, but is not limited thereto.

In an aspect of the present invention, the pharmaceutical composition may further include a drug for increasing an anti-cancer effect. The drug includes an anti-cancer agent, a stimulator of interferon genes (STING) agonist, a TGF beta inhibitor, a drug for inhibiting T helper 17 cell (Th17) immune response, a drug for inhibiting production or activity of interleukin (IL)-6, a drug for inhibiting production or activity of vascular endothelial growth factor (VEGF), and a drug for inhibiting signal transduction of signal transducer and activator of transcription 3 (STAT3), but is not limited thereto. An example of the drug for inhibiting the Th17 immune response may include aspirin, and an example of the drug for inhibiting production or activity of VEGF may include a drug for inhibiting signal transduction by VEGF receptor.

In an aspect of the present invention, the membrane of the bacterial extracellular vesicle may further include components other than the cell membrane of the bacteria.

The components other than the cell membrane may include a targeting material, a cell membrane fusion material (fusogen), cyclodextrin, and polyethylene glycol. In addition, the components other than the cell membrane may be added by various methods, including chemical modification of the cell membrane.

For example, the membrane components of the bacterial extracellular vesicles may be modified by a chemical method using a thiol group (-SH) or an amine group (-NH₂). The membrane components of the bacterial extracellular vesicles may also be chemically modified by chemically binding polyethyleneglycol to the bacterial extracellular vesicles.

Accordingly, the bacterial extracellular vesicles according to the present invention may further be prepared by a step including chemical modification of the membrane components.

The bacterial extracellular vesicles according to the present invention may be isolated from a bacterial culture according to various methods known in the art. A type of technology for isolating the extracellular vesicles from the bacterial culture is not particularly limited, and for example, may use methods, such as ultracentrifugation, density gradient ultracentrifugation, ultrafiltration, size-exclusion chromatography, ion exchange chromatography, immunoaffinity capture, microfluidics-based isolation, aqueous two-phase system or precipitation.

In the present invention, the "immune checkpoint" is a generic term for proteins involved in inducing stimulatory or inhibitory signals of immune responses on the surface of immune cells, and manipulates the cancer cells so as to prevent the stimulation of the immune responses and inhibition of cancer cells from progressing well to evade the surveillance of the immune system. Preferably, the immune checkpoint protein may include programmed cell death-1 (PD1), programmed cell death-ligand 1 (PDL1), programmed cell death-ligand 2 (PDL2), cluster of differentiation 27 (CD27), cluster of differentiation 28 (CD28), cluster of differentiation 70 (CD70), cluster of differentiation 80 (CD80), cluster of differentiation 86 (CD86), T cell immunoreceptor with Ig and ITIM domains (TIGIT), cluster of differentiation 137 (CD137), cluster of differentiation 276 (CD276), killer-cell immunoglobulin-like receptors (KIRs), lymphocyte-activation gene 3 (LAG3), tumor necrosis factor receptor superfamily, member 4 (TNFRSF4), glucocorticoid-induced TNFR-related protein (GITR), glucocorticoid-induced TNFR-related protein ligand (GITRL), 4-IBB ligand (4-1BBL), cytotoxic T lymphocyte associated antign-4 (CTLA4), adenosine A2A receptor (A2aR), V-set domain-containing T-cell activation inhibitor 1 (VTCN1), B- and T-lymphocyte attenuator (BTLA), indoleamine 2,3-dioxygenase (IDO), T-cell immunoglobulin domain and mucin-domain containing-3 (TIM3), V-domain Ig suppressor of T cell activation (VISTA), and killer cell lectin-like receptor subfamily A (KLRA), preferably PDL1, TIGIT, CD80 or CTLA4.

In the present invention, the "immune checkpoint inhibitor" is an antagonist targeting the immune checkpoint protein, which enhances a protein that stimulates immune responses or blocks a protein that inhibits the immune response to exhibit anti-cancer effects by the immune responses.

In an aspect of the invention, the immune checkpoint inhibitor may be a protein or peptide such as a soluble fusion protein. In an aspect, the protein includes a receptor/ligand binding domain (e.g. extracellular domain) of PD1, PDL1, PDL2, CD27, CD28, CD70, CD80, CD86, TIGIT, CD137, CD276, KIRs, LAG3, TNFRSF4, GITR, GITRL, 4-1BBL, CTLA4, A2aR, VTCN1, BTLA, IDO, TIM3, VISTA, or KLRA. In an aspect, the receptor/ligand binding domain may be fused to an immunoglobulin Fc domain. Such fusion protein may be prepared by standard recombinant DNA technique (e.g., see Current Protocols in Molecular Biology, Ausubel et al., eds., John Wiley & Sons: 1992). Moreover, it is already commercially available to use many expression vectors encoding a fusion moiety.

In another aspect of the present invention, the immune checkpoint inhibitor may be an antibody or an antigen-binding fragment thereof which binds to an immune checkpoint protein (e.g., PD1, PDL1, PDL2, CD27, CD28, CD70, CD80, CD86, TIGIT, CD137, CD276, KIRs, LAG3, TNFRSF4, GITR, GITRL, 4-1BBL, CTLA4, A2aR, VTCN1, BTLA, IDO, TIM3, VISTA, or KLRA) to be inhibited. In the present invention, the "antibody" may refer to both an intact antibody and an antigen-binding fragment thereof. The antibody includes monoclonal antibodies, polyclonal antibodies, chimeric antibodies, humanized antibodies, human antibodies, multi-specific antibodies (e.g., bi-specific antibodies), single-chain antibodies and antigen-binding antibody fragments. The antigen-binding fragment of the antibody refers to one or more fragments of an antibody that is able to bind an antigen. Examples of antigen-binding fragments include Fab, Fab', F(ab')2, Fv, scFv, Fv linked with disulfide bonds, Fd, diabodies, single chain antibodies, camelid antibodies, isolated CDR-H3, and other antibody fragments that have at least a portion of a variable region of an intact antibody. Such antibody fragments may be obtained using conventional recombinant and/or enzymatic techniques and screened for antigen-binding in the same manner as intact antibodies.

In another aspect of the present invention, the immune checkpoint inhibitor may be inhibitory nucleic acids (e.g., siRNA molecule, shRNA molecule, anti-sense RNA), which specifically bind to mRNA coding the immune checkpoint protein (e.g., PD1, PDL1, PDL2, CD27, CD28, CD70, CD80, CD86, TIGIT, CD137, CD276, KIRs, LAG3, TNFRSF4, GITR, GITRL, 4-1BBL, CTLA4, A2aR, VTCN1, BTLA, IDO, TIM3, VISTA, or KLRA). Inhibitory nucleic acid molecules may be prepared by chemical synthesis, *in vitro* transcription, or cleavage of long dsRNAs by Rnase III or Dicer. The inhibitory nucleic acid molecules may be delivered to cells *in vitro* or to a tumor or hypoxic tissue of a mammal *in vivo.* All typical delivery means known in the art may be used. For example, interfering RNAs may be delivered systemically using, for example, a method and a composition described in PCT Application No. PCT/US2009/036223, each of which is cited entirely by the reference. In a specific embodiment, the inhibitory nucleic acids are delivered topically. For example, when the inhibitory nucleic acids described herein are used to treat cancer, the delivery to the tumor may be performed by injection into tumor as described in, for example, Takahashi et al., Journal of Controlled Release 116:90-95 (2006) and Kim et al., Journal of Controlled Release 129:107-116 (2008), each of which is cited entirely by the reference.

In an aspect of the present invention, the immune checkpoint inhibitor may be selected from the group consisting of Durvalumab, Atezolizumab, Avelumab, Tremelimumab, Ipilimumab, Pembrolizumab, Nivolumab, Pidilizumab, BMS986016, Cemiplimab and Lirilumab, but is not limited thereto.

Compared with general anti-cancer cytotoxic drugs, the immune checkpoint inhibitors have less adverse effects, such as vomiting and hair loss, and have high therapeutic effects. As they use the immune response systems with good memory, the therapeutic effects last for a long time after stopping drug administration. However, it is not known whether their anti-cancer effects are enhanced by combining with the bacterial extracellular vesicles. Accordingly, synergic effects of cancer treatment by combining the immune checkpoint inhibitors and the bacterial extracellular vesicles may be a technical feature of the present invention.

In an aspect of the present invention, the bacterial extracellular vesicles and the immune checkpoint inhibitors may be co-administered simultaneously, sequentially or separately. In the present invention, the "simultaneously" means that two agents are administered at the same time, and "sequentially" means that one agent is administered within 5 minutes, within 10 minutes, or within several hours after the other agent is administered. However, the half-life in circulation of the first administered agent is provided so that the two agents are both simultaneously present in a therapeutically effective dose. In addition, simultaneous, sequential, or separate administration methods are not limited to one time, and these administration methods may be administered repeatedly or in combination.

In an aspect, the bacterial extracellular vesicles and the immune checkpoint inhibitors may be included in the same composition together with a pharmaceutically acceptable carrier, an excipient, and/or a diluent.

In another aspect, the bacterial extracellular vesicles and the immune checkpoint inhibitors may be provided in separate pharmaceutical forms or kit-of-parts.

In an aspect of the present invention, the cancer may be selected from the group consisting of gastric cancer, lung cancer, non-small cell lung cancer, breast cancer, ovarian cancer, hepatic cancer, bronchial cancer, nasopharyngeal cancer, laryngeal cancer, pancreatic cancer, colorectal cancer, bladder cancer, colon cancer, cervical cancer, bone cancer, non-small cell bone cancer, hematologic malignancy, skin cancer, head or neck cancer, uterine cancer, rectal cancer, perianal cancer, colon cancer, fallopian tube cancer, endometrial cancer, vaginal cancer, cancer of the vulva, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, renal or ureteral cancer, renal cell carcinoma, Renal pelvic cancer, salivary gland cancer, sarcoma cancer, pseudomyxoma, hepatoblastoma, testicular cancer, glioblastoma, lip cancer, germ cell tumor of ovary, basal cell carcinoma, multiple myeloma, gallbladder cancer, choroidal melanoma, ampullar of vater cancer, peritoneal cancer, tongue cancer, small cell cancer, pediatric lymphoma, neuroblastoma, duodenal cancer, ureteral cancer, astrocytoma, meningioma, renal pelvis cancer, vulvar cancer, thymus cancer, central nervous system (CNS) tumor, primary central nervous system lymphoma, spinal cord tumor, brainstem glioma, and pituitary adenoma, but is not limited thereto.

The pharmaceutical composition according to the present invention may include only the bacterial extracellular vesicles and the immune checkpoint inhibitors, or may be formulated in a suitable form together with a pharmaceutically acceptable carrier, and further include excipients or diluents. The carrier includes all kinds of solvents, dispersion media, oil-in-water or water-in-oil emulsions, aqueous compositions, liposomes, microbeads and microsomes.

The composition of the present invention may be administered to mammals including humans by any method. For example, the composition may be administered orally or parenterally. The parenteral administration method is not limited thereto, but may include intravenous, intramuscular, intraarterial, intramedullary, intrathecal, intracardiac, transdermal, subcutaneous, intraperitoneal, intranasal, intestinal, topical, sublingual, or rectal administration.

The pharmaceutical composition of the present invention may be formulated as a preparation for oral administration or parenteral administration according to the route of administration as described above.

The total effective dose of the composition of the present invention may be administered to a patient in a single dose, or may be administered in multiple doses for a long period of time according to a fractionated treatment protocol. In the pharmaceutical composition of the present invention, the content of the active ingredient may vary depending on the severity of diseases. Preferably, the preferred total dose of the pharmaceutical composition of the present invention may be about 0.01 µg to 10,000 mg, most preferably 0.1 µg to 500 mg per 1 kg of patient's body weight per day. However, the effective dose of the pharmaceutical composition is determined by consiering various factors, including the age, body weight, health conditions, and gender of a patient, the severity of a disease, diet, and excretion rate as well as a formulation method, a route of administration and the number of treatment times, those skilled in the art may determine a suitable effective dose of the composition of the present invention by considering these points. The pharmaceutical composition according to the present invention is not particularly limited to the formulation, the administration route, and the administration method, as long as the effects of the present invention are shown.

In the present invention, the "target cells" collectively refer to cells involved in binding to bacterial extracellular vesicles, surface-modified extracellular vesicles, or immune checkpoint inhibitors, or those involved in inducing physiologically stimulatory or inhibitory signals. The target cells include cancer cells, immune cells (T lymphocytes, B lymphocytes, NK cells, NKT cells, macrophages, dendritic cells, monocytes, neutrophils, eosinophils, basophils, mast cells, myeloid-derived suppressor cells, etc.), endothelial cells, epithelial cells, and fibroblasts, but are not limited thereto.

The present invention provides the use of bacterial extracellular vesicles and immune checkpoint inhibitors for the preparation of a cancer therapeutic agent.

The present invention provides a cancer treatment method including administering an effective amount of a composition including the bacterial extracellular vesicles and the immune checkpoint inhibitors as active ingredients to a subject in need thereof.

The 'effective dose' of the present invention means an amount which exhibits effects of improving, treating, detecting, and diagnosing of cancer, or inhibiting or reducing the progression of the cancer when administered to the subject, and the 'subject' may be animals, preferably, mammals, particularly animals including humans and may also be cells, tissues, and organs derived from animals. The subject may be a patient requiring the effects.

The 'treatment' of the present invention comprehensively refers to improving a cancer or symptoms caused by the cancer, and may include treating or substantially preventing the cancer, or improving the conditions thereof and includes alleviating, treating or preventing a symptom or most of the symptoms derived from the cancer, but is not limited thereto.

The term "comprising" used herein is used in the same meaning as "including" or "characterized by", and does not exclude additional ingredients or steps of the method, which are not specifically mentioned in the composition or the method according to the present invention. In addition, the term "consisting of" means excluding additional elements, steps or ingredients, unless otherwise described. The term "essentially consisting of" means including materials or steps which do not substantially affect basic properties thereof in addition to the described materials or steps within the range of the composition or the method.

### ADVANTAGEOUS EFFECTS

According to the present invention, the composition including the bacterial extracellular vesicles and immune checkpoint inhibitors, either a single type or the combination of two or more types, as active ingredients exhibits a synergistic effect in cancer treatment compared to monotherapy using each agent. The composition also exhibits an effect of reducing adverse effects caused by high-dose administration of the single agents. As a result, the composition can be very usefully utilized for development of cancer therapeutic agents by providing the method for enhancing the cancer treatment effect.

### DESCRIPTION OF DRAWINGS

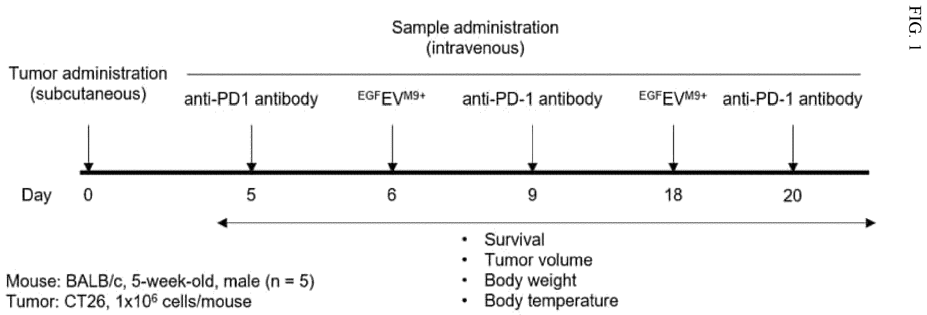

FIG. 1 illustrates an experimental schedule for co-administration of anti-PD1 antibodies and native extracellular vesicles (^{EGF}EV^{M9+}) to mice transplanted with colorectal cancer cells (CT26).

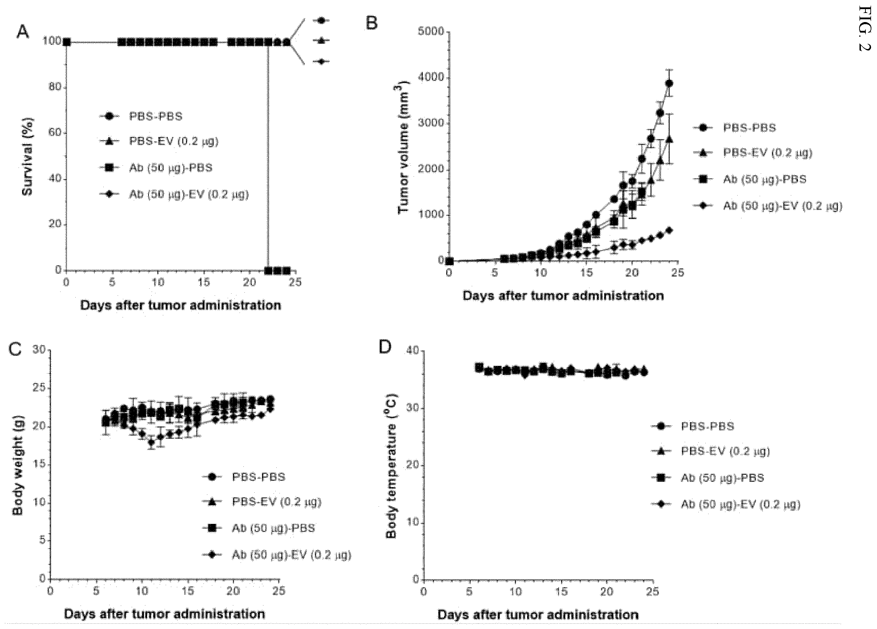

FIG. 2, including FIGS. 2A and 2B, illustrates results of measuring the survival rates, tumor volumes, and changes in body weights as well as body temperatures of the animal during a period of co-administration of anti-PD1 antibodies and native extracellular vesicles (^{EGF}EV^{M9+}) to mice transplanted with colorectal cancer cells (CT26).

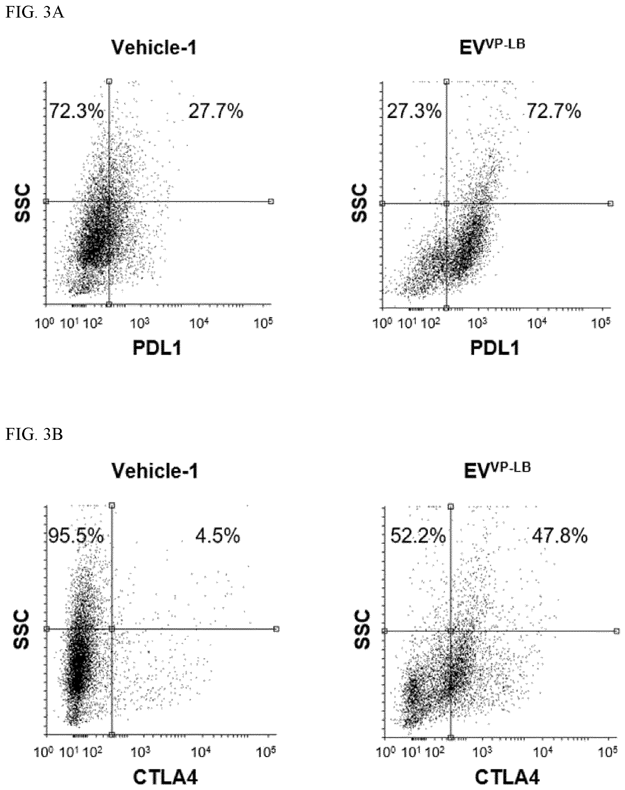

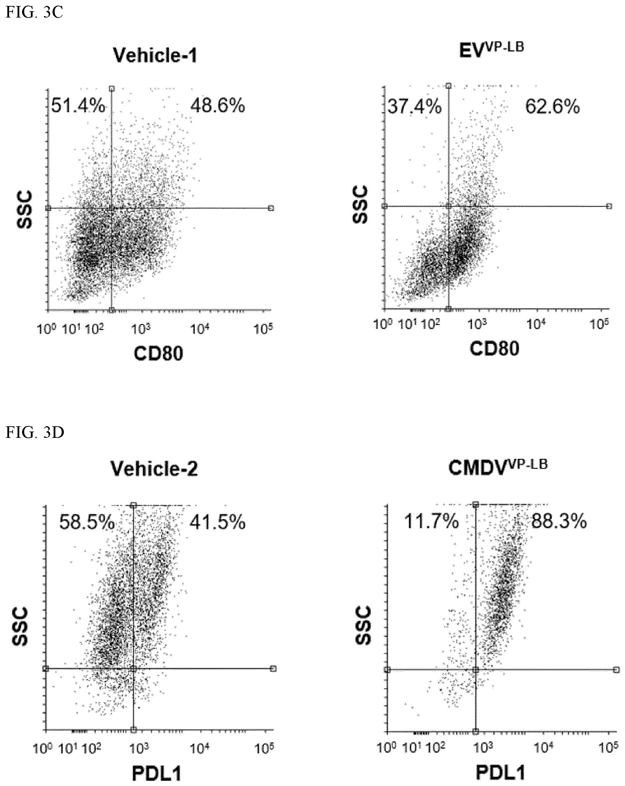

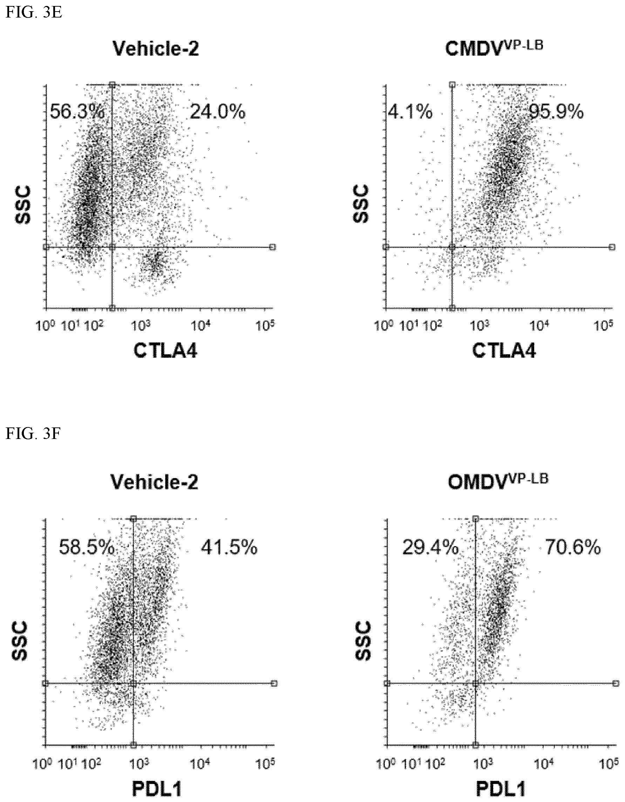

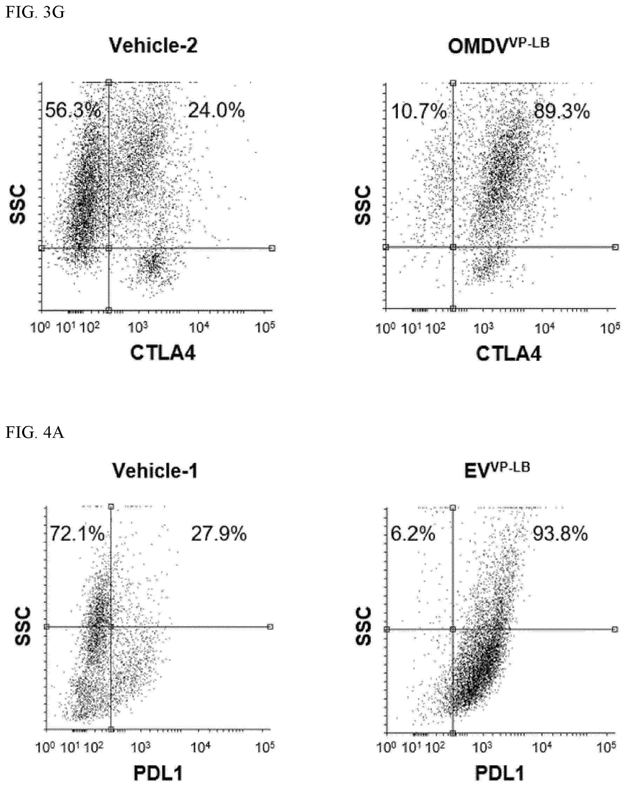

FIG. 3, including FIGS. 3A to 3G, illustrates results of confirming changes in expression of immune checkpoint proteins (PDL1, CTLA4, and CD80) by analyzing cells present in tumor microenvironments by flow cytometry after administering native or artificial extracellular vesicles EV^{VP-LB}, CMDV^{VP-LB} or OMDV^{VP-LB} to mice transplanted with bladder cancer cells (MB49).

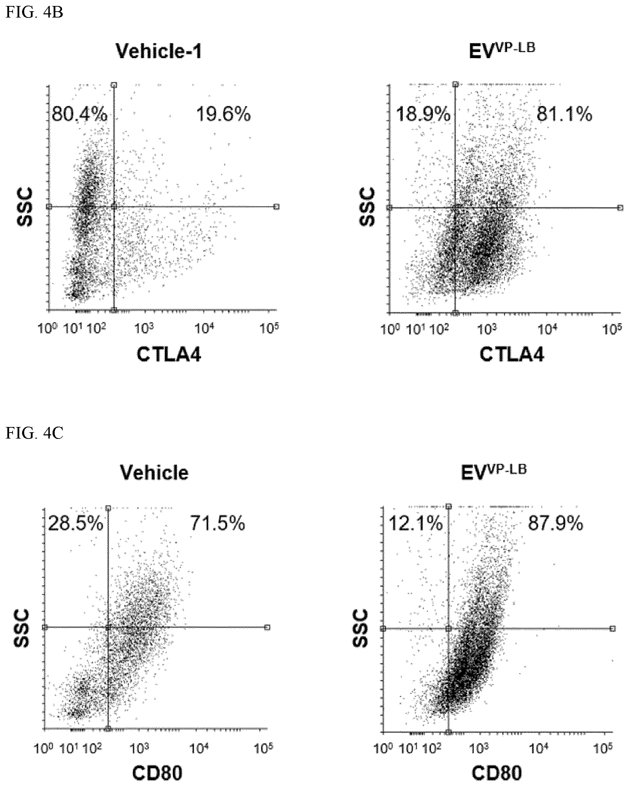

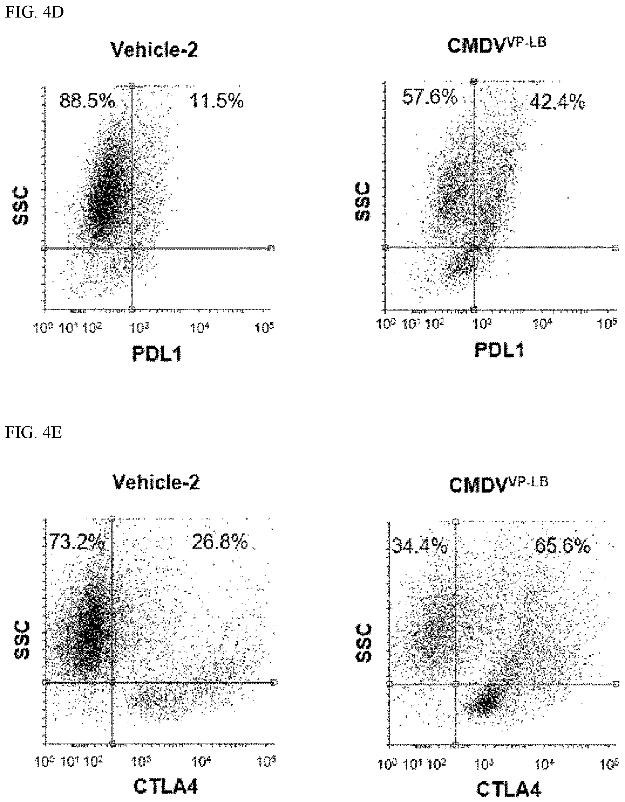

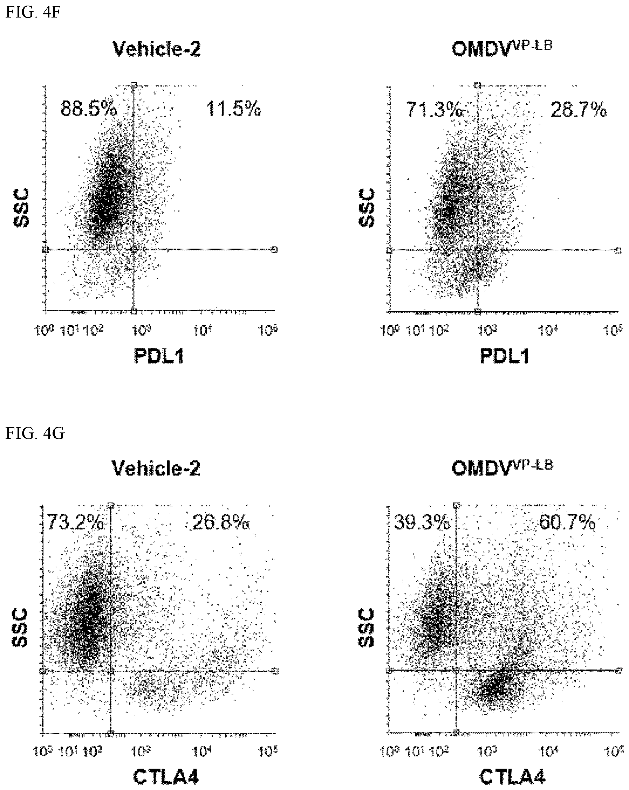

FIG. 4, including FIGS. 4A to 4G, illustrates results of confirming changes in expression of immune checkpoint proteins (PDL1, CTLA4, and CD80) by analyzing cells present in tumor microenvironments by flow cytometry after administering native or artificial extracellular vesicles EV^{VP-LB}, CMDV^{VP-LB} or OMDV^{VP-LB} to mice transplanted with colorectal cancer cells (CT26).

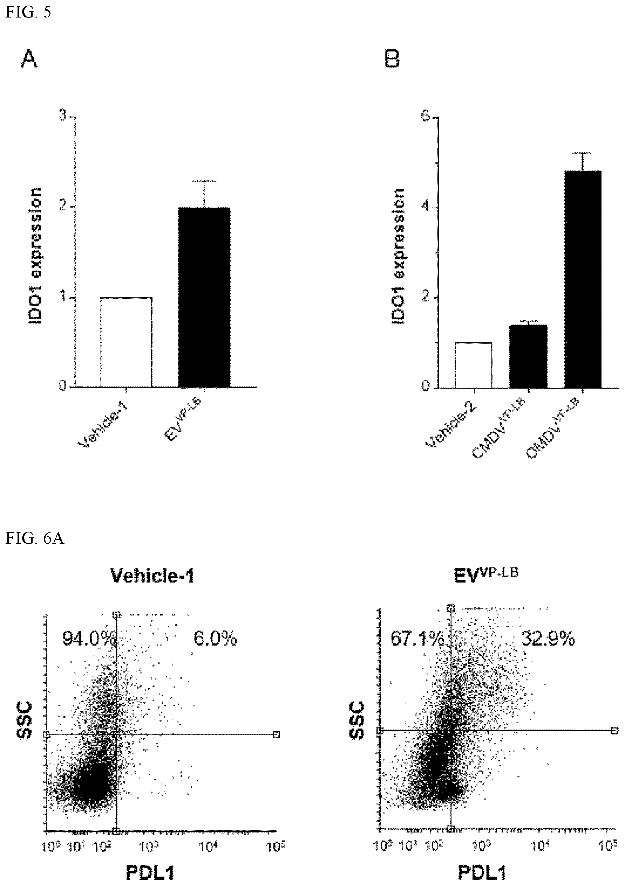

FIG. 5 illustrates results of confirming changes in expression of an immune checkpoint protein (IDO1) by analyzing cells present in tumor microenvironments by qRT-PCR after administering native or artificial extracellular vesicles EV^{VP-LB}, CMDV^{VP-LB} or OMDV^{VP-LB} to mice transplanted with bladder cancer cells (MB49).

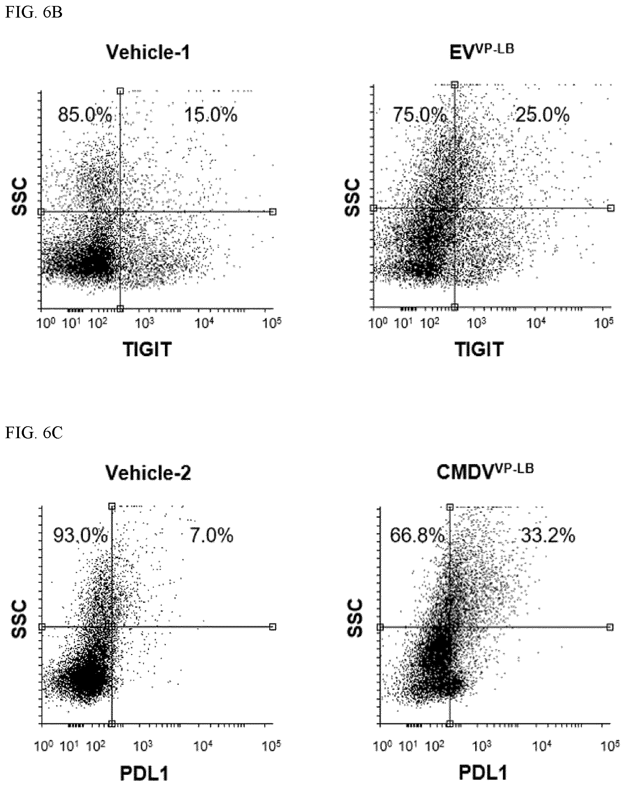

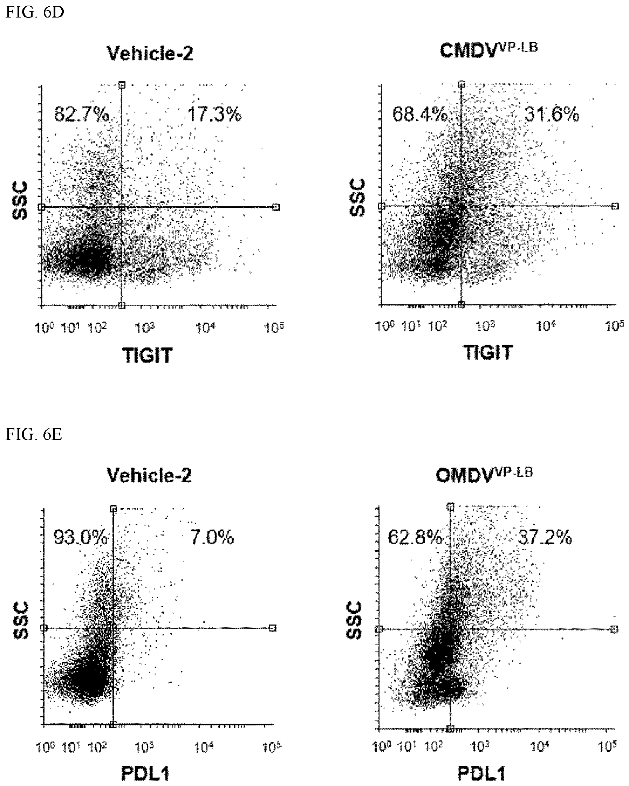

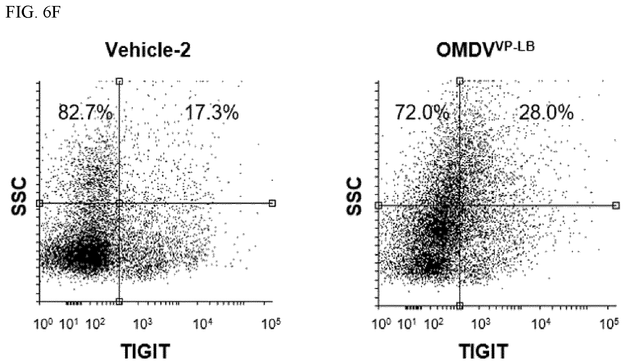

FIG. 6, including FIGS. 6A to 6F, illustrates results of confirming changes in expression of immune checkpoint proteins (PDL1 and TIGIT) by analyzing cells by flow cytometry after treating native or artificial extracellular vesicles EV^{VP-LB}, CMDV^{VP-LB} or OMDV^{VP-LB} to splenocytes extracted from mice transplanted with mouse colorectal cancer cells (CT26).

### MODES FOR THE INVENTION

Hereinafter, the present invention will be described in details by the following Examples. However, the following Examples are just illustrative of the present invention, and the contents of the present invention are not limited to the following Examples.

### Example 1: Effects of in vivo co-administration of bacterial extracellular vesicles (EV) and immune checkpoint inhibitors

### Experiment method

### Preparation of native extracellular vesicles

As shown in Table 1 below, a medium (M9+) was prepared in which vitamins and trace elements were added to M9 chemically defined medium. *E. coli ΔmsbB*, in which lipopolysaccharide was attenuated, was transformed with pHCE-prsA-EGF vector expressing a fusion protein of human EGF and a bacterial inner membrane protein PrsA, resulting in *E. coli ΔmsbB* prsA-EGF. The transformed bacteria were cultured using M9+ medium.

**[Table 1]**

| Component | | Concentration |
|---|---|---|
| **Salts and glucose** | | |
| | Na₂HPO₄ •2H₂O | 42.3 mM |
| | KH₂PO₄ | 22.0 mM |
| | NH₄Cl | 9.4 mM |
| | NaCl | 8.6 mM |
| | MgSO₄ • 7H₂O | 1.0 mM |
| | CaCl₂ • 2H₂O | 0.3 mM |
| | Glucose | 0.4% |

| **Vitamins** | | |
|---|---|---|
| | Thiamine | 3.0 µM |
| | Biotin | 4.1 µM |

| **Trace elements** | | |
|---|---|---|
| | EDTA | 0.2 mM |
| | FeCl₃ | 30.7 µM |
| | ZnCl₂ | 6.2 µM |
| | CuCl₂·2H₂O | 0.8 µM |
| | CoCl₂ • 6H₂O | 0.4 µM |
| | H₃BO₃ | 1.6 µM |
| | MnCl₂·4H₂O | 81.0 nM |

The culture was centrifuged twice at 6,000 × g at 4°C for 20 minutes to remove bacteria, and the supernatant was filtrated with a 0.45 µm pore-sized filter. Then, the filtrate was concentrated in 50-folds using a membrane capable of removing proteins of which molecular weights are less than 100 kDa. After the concentrate was filtrated with a 0.22 µm pore-sized filter, the filtrate was ultracentrifuged at 4°C at 150,000 × g for 3 hours. The pellet was suspended in 2.5 mL of 50% iodixanol, put into a 5 mL volume of ultracentrifuge tube, and then 1.5 mL of 40% iodixanol and 1.25 mL of 10% iodixanol were sequentially placed thereon. Thereafter, pure bacterial extracellular vesicles were obtained from a layer between 10% iodixanol and 40% iodixanol by ultracentrifugation at 200,000 × g at 4°C for 2 hours.

Finally, the isolated bacterial extracellular vesicles were native extracellular vesicles, and termed as ^{EGF}EV^{M9+}.

### Combinatorial treatment of bacterial extracellular vesicles and immune checkpoint inhibitors

To evaluate anti-cancer activity of combination of bacterial extracellular vesicles and immune checkpoint inhibitors, mouse colorectal cancer cells (CT26, 1 × 10⁶ cells/head) were subcutaneously administered to mice (BALB/c, male, 5 weeks old). At five days after the tumor cell administration, the mice were divided into four experimental groups, each consists of five mice (see Table 2). The purified bacterial extracellular vesicles and immune checkpoint inhibitors were administered alone or in combination to each group, and the immune checkpoint inhibitors used in the experiment were anti-PD1 antibodies (InVivoMAb anti-mouse PD1; Bio X Cell (Lebanon, NH, USA), Cat# BE0273). First, at 5 days after tumor cell administration, each group was administered with PBS or an immune checkpoint inhibitor (Ab).

At 6 days after tumor cell administration, each group was administered with PBS or ^{EGF}EV^{M9+} (EV). At 9 days after tumor cell administration, each group was administered with PBS or Ab, and then at 18 days after tumor cell administration, each group was administered with PBS or Ab again. Finally, at 20 days after tumor cell administration, each group was administered with PBS or Ab (FIG. 1).

Next, the survival of the mice was monitored daily from 6 days to 24 days after tumor cell administration for each experimental group, and tumor volumes were measured. The tumor volumes (V) were calculated using an equation V = l × s²/2, by measuring the longest length (l) and the length (s) perpendicular thereto.

In addition, changes in body weights and body temperatures of mice in each experimental group were continuously observed daily from 6 days to 24 days after tumor cell administration.

**[Table 2]**

| Group # | PBS or Ab | PBS or EV |
|---|---|---|
| 1 | PBS | PBS |
| 2 | PBS | EV (0.2 µg) |
| 3 | Ab (50 µg) | PBS |
| 4 | Ab (50 µg) | EV (0.2 µg) |

### Experimental Results

The experimental results were shown in FIG. 2.

As shown in FIG. 2, in PBS-PBS control group, PBS-EV (0.2 µg), and Ab (50 µg)-EV (0.2 µg) experimental groups, there were no mice died, and in Ab (50 µg)-PBS experimental group, all mice died at day 22 (FIG. 2A).

Compared to PBS-PBS control group, the final tumor volumes were significantly reduced in PBS-EV (0.2 µg) and Ab (50 µg)-EV (0.2 µg) experimental groups, and Ab (50 µg)-PBS experimental group could not be compared as all the mice in the group were finally dead. In addition, compared with that of PBS-EV (0.2 µg) experimental group, the final tumor volume was significantly reduced in Ab (50 µg)-EV (0.2 µg) experimental group (FIG. 2B).

Thus, compared with those of experimental groups administered with EV or Ab alone, tumor growth in the group administered with Ab and EV in combination was significantly reduced, confirming the synergistic effects. Especially, all mice were dead at day 22 in the experimental group administered with Ab alone, but no mice were dead until the end of experiment in the experimental group administered with Ab and EV in combination, suggesting that toxicity could be reduced upon combination of two agents.

There were no significant changes in body weights and body temperatures in the control group and all the experimental groups during the entire experimental period (FIGs. 2C and 2D).

### Example 2: Preparation and characterization of bacterial extracellular vesicles

An additional experiment was performed to conduct an experiment to find the scientific basis of the synergistic effect (the synergistic effect of combining the bacterial extracellular vesicles and the immune checkpoint inhibitors) confirmed in Example 1 above. The bacterial extracellular vesicles used in the additional experiment were prepared by the following method.

### Preparation of native extracellular vesicles

The native extracellular vesicles used in the experiment were derived from *E*. *coli* BL21 (DE3) *ΔmsbB* and were prepared according to Example 4 of KR10-2021-0080108 filed in Korea on June 21, 2021, and the finally isolated native extracellular vesicles were termed as EV^{VP-LB}.

### Preparation of artificial extracellular vesicles

Artificial extracellular vesicles used in the experiment included cell membrane-derived vesicles (CMDVs) and outer membrane-derived vesicles (OMDVs). To isolate the CMDVs and OMDVs, *E. coli* BL21 (DE3) *ΔmsbB*-AmCyan was cultured using vegetable peptone-based lysogeny broth (VP-LB). The culture was centrifuged (6,000 × g for 15 minutes), and the supernatant was discarded to obtain bacterial cell pellets, which were suspended in vehicle-2 (10 mM Tris-HCl (pH 8.0), 5% sucrose). Bacterial cells were lysed by passing the suspension through a microfluidic device (10,000 psi for 5 times), and centrifuged (6,000 × g, 20 minutes) to obtain the supernatant containing the lysed cell fragments. The supernatant was concentrated in 3-folds using a tangential flow filter (Tangential flow filtration: > 500 kDa filter). The concentrate was divided into halves, and the half was used to isolate CMDVs and the other half was used to isolate OMDVs.

To prepare CMDVs, the previously prepared concentrate was treated with benzonase (final concentration: 50 units/mL) together with MgCl₂ (final concentration: 2 mM) at 37°C for 30 minutes. Thereafter, vehicle-2 was added in 4-fold volume of the concentrate, and concentrated in 5-folds using a tangential flow filter (> 500 kDa). Subsequently, CMDVs were isolated through density gradient ultracentrifugation (sample 6.5 mL, 0.8 M sucrose 3.0 mL, 2.5 M sucrose 3.0 mL; 200,000 × g, for 2 hours).

To prepare OMDVs, the previously prepared concentrate was treated with sarkosyl (final concentration: 1%) at room temperature for 30 minutes. Thereafter, vehicle-2 was added in 4-fold volume of the concentrate, concentrated in 5-folds using a tangential flow filter (> 500 kDa). Next, the concentrate was treated with benzonase (final concentration: 50 units/mL) together with MgCl₂ (final concentration: 2 mM) at 37°C for 30 minutes. Thereafter, vehicle-2 was added in 4-fold volume of the concentrate and concentrated in 25-folds using a tangential flow filter (> 500 kDa). Subsequently, OMDVs were isolated through density gradient ultracentrifugation (sample 6.5 mL, 0.8 M sucrose 3.0 mL, 2.5 M sucrose 3.0 mL; 200,000 × g, for 2 hours).

The finally isolated CMDVs and OMDVs were termed as CMDV^{VP-LB} and OMDV^{VP-LB}, respectively.

### Characterization of bacterial extracellular vesicles

The isolated bacterial extracellular vesicles were characterized, as previously described in the literature (Biomaterials. 113:68-79, 2017). First, the size distribution was measured by dynamic light scattering using Zetasizer Nano ZS (Malvern Instruments). In addition, the protein concentration and total protein amount were measured using Bradford assay, and the particle concentration and total particle number were measured by nanoparticle tracking analysis using LM10-HS (Malvern Instruments).

The results of characterization of bacterial extracellular vesicles were shown in Table 3 below.

**[Table 3]**

| | Size (nm) | Concentration | | Total amount | |
|---|---|---|---|---|---|
| | | Protein (mg/mL) | Particle (x10¹²/mL) | Protein (mg) | Particle (x10¹²) |
| CMDV^{VP-LB} | 156.8 ± 61.2 | 5.3 | 28.7 | 5.3 | 28.7 |
| OMDV^{VP-LB} | 127.6 ± 42.8 | 2.3 | 24.6 | 2.5 | 27.3 |

### Example 3: Analysis of changes in tumor microenvironment (TME) upon administration of bacterial extracellular vesicles

### Experiment method

Mouse bladder cancer cells (MB49: administered to C57BL/6; 1×10⁶ cells/head) and colon cancer cells (CT26: administered to BALB/c; 1 × 10⁶ cells/head) were subcutaneously administered to mice (C57BL/6, male, 7 weeks old and BALB/c, female, 7 weeks old), and tumors were grown for 7 days. Mice were divided into 5 mice per group and EV^{VP-LB}, CMDV^{VP-LB}, or OMDV^{VP-LB} was intratumorally administered as shown in Table 2 below. At 24 hours after administration of EV^{VP-LB}, CMDV^{VP-LB}, or OMDV^{VP-LB}, the tumor tissues were extracted from each group, and changes in the tumor microenvironment were analyzed by flow cytometry or real-time RT-PCR.

**[Table 4]**

| Group # | Sample |
|---|---|
| 1 | Vehicle-1 (for EV^{VP-LB}) |
| 2 | EV^{VP-LB} 5 µg |
| 3 | Vehicle-2 (for CMDV^{VP-LB} and OMDV^{VP-LB}) |
| 4 | CMDV^{VP-LB} 10 µg |
| 5 | OMDV^{VP-LB} 10 µg |

| | |
|---|---|
| Vehicle -1: 10 mM L-histidine (pH 7.4), 107 mM NaCl, 2% sucrose Vehicle-2: 10 mM Tris-HCl (pH 8.0), 5% sucrose | |

### (1) Flow cytometry analysis of tumor microenvironment

A method described in the literature (Methods Mol Biol. 1458:95-110, 2016) was used to prepare cells for flow cytometry analysis. Anti-PDL1 and anti-CTLA4 from BD Biosciences Co., Ltd. were used as antibodies, and LSR Fortessa (5 Laser) model from BD Biosciences Co., Ltd. was used as an analysis device.

### (2) Real-time RT-PCR analysis of tumor microenvironment

A method described in the literature (Invitrogen, TRIzol^{™} Reagent User guide) was used to prepare RNAs for real-time RT-PCR analysis, and the prepared RNA was prepared as cDNA using GoScirpt^{™} Reverse Transcriptase Kit (Promega). GAPDH was used as an internal control, real-time PCR was performed on StepOnePlus Real-Time PCR System (Applied Biosystems), and primers required for real-time PCR were purchased from Bioneer.

### Experimental Results

### (1) Flow cytometry analysis of tumor microenvironment

Changes in expression of immune checkpoint proteins PDL1, CTLA4, and CD80 were assessed by flow cytometry on cells present in tumor microenvironment (FIGs. 3A to 3G and FIGs. 4A to 4G).

First, the analysis was performed in the cases where extracellular vesicles were administered to the mice which had been administered with mouse bladder cancer cells (MB49). Compared to the control group, the expression of PDL1 increased by 45.0% (27.7% → 72.7%), the expression of CTLA4 increased by 43.3% (4.5% → 47.8%), and the expression of CD80 increased by 14.0% (48.6% → 62.6%) in EV^{VP-LB}-administered group (FIGs. 3A to 3C). The expression of PDL1 increased by 46.8% (41.5% → 88.3%) and the expression of CTLA4 increased by 71.9% (24.0% → 95.9%) in CMDV^{VP-LB}-administered group (FIGs. 3D and 3E). Meanwhile, the expression of PDL1 increased by 29.1% (41.5% → 70.6%) and the expression of CTLA4 increased by 65.3% (24.0% → 89.3%) in OMDV^{VP-LB}-administered group (FIGs. 3F and 3G).

In addition, the analysis was also conducted in the cases where extracellular vesicles were administered to the mice which had been administered with mouse colorectal cancer cells (CT26). Compared to the control group, the expression of PDL1 increased by 65.9% (27.9% → 93.8%), the expression of CTLA4 increased by 61.5% (19.6% → 81.1%), and the expression of CD80 increased by 16.4% (71.5% → 87.9%) in EV^{VP-LB}-administered group (FIGs. 4Ato 4C). The expression of PDL1 increased by 30.9% (11.5% → 42.4%) and the expression of CTLA4 increased by 38.8% (26.8% → 65.6%) in CMDV^{VP-LB}-administered group (FIGs. 4D and 4E). Meanwhile, the expression of PDL1 increased by 17.2% (11.5% → 28.7%) and the expression of CTLA4 increased by 33.9% (26.8% → 60.7%) in OMDV^{VP-LB}-administered group (FIGs. 4F and 4G).

Thus, the proportion of cells expressing immune checkpoint proteins PDL1, CTLA4, and CD80 increased in the mouse tumor microenvironment when bacterial extracellular vesicles were administered.

The above results may scientifically explain a synergistic effect by combining the bacterial extracellular vesicles of Example 1 and PDL1. Furthermore, the anti-cancer effect of the bacterial extracellular vesicles can be expected to increase when co-administered with materials capable of inhibiting CD80 and CTLA4 in addition to a PDL1 inhibitor.

### (2) Real-time RT-PCR analysis result of tumor microenvironment

The results of measuring changes in the expression of an immune checkpoint protein IDO1 by real-time RT-PCR were shown in FIG. 5.

As shown in FIG. 5, the expression of the immune checkpoint protein IDO1 increased in the mouse tumor microenvironment when EV^{VP-LB}, CMDV^{VP-LB}, or OMDV^{VP-LB} was administered. Therefore, the anti-cancer effect of the bacterial extracellular vesicles can be expected to increase when co-administered with an IDO inhibitor.

### Example 4: Analysis of effect of bacterial extracellular vesicles on splenocytes

The spleen is a secondary lymphoid organ, where various immune cells exist and affect systemic immune responses. It is assumed that the spleen affects and is affected with tumor tissues if the tumor exists. Therefore, important clues for cancer treatment can be provided from observing changes in the splenocytes, obtained from tumor-bearing mice, treated with bacterial extracellular vesicles.

### Experiment method

Mice (BALB/c, female, 7 weeks old) were subcutaneously administered with colorectal cancer cells (CT26, 1×10⁶ cells/head). At 14 days after tumor cell administration, the spleen was extracted, and single splencoytes were isolated. The isolated single splenocytes were treated with EV^{VP-LB}, CMDV^{VP-LB}, or OMDV^{VP-LB} (1 µg/mL each) for 24 hours, and flow cytometry was performed. A method described in the literature (Methods Mol Biol. 1458:95-110, 2016) was used to prepare cells for flow cytometry analysis. Anti-PDL1 and anti-TIGIT from BD Biosciences Co., Ltd. were used as antibodies, and LSR Fortessa (5 Laser) model from BD Biosciences Co., Ltd. was used as an analysis device.

### Experimental Results

Mice were administered with mouse colorectal cancer cells (CT26), and the splenocytes isolated from the mice were treated with bacterial extracellular vesicles and analyzed by flow cytometry. As a result, the expression of immune checkpoint proteins PDL1 and TIGIT were changed (FIGs. 6A to 6F).

As shown in FIGs. 6A to 6F, when the splenocytes were treated with EV^{VP-LB}, the expression of PDL1 increased by 26.9% (6.0% → 32.9%) and the expression of TIGIT increased by 10.0% (15.0% → 25.0%) (FIGs. 6A and 6B). When treated with CMDV^{VP-LB}, the expression of PDL1 increased by 26.2% (7.0% → 33.2%) and the expression of TIGIT increased by 14.3% (17.3% → 31.6%) (FIGs. 6C and 6D). Meanwhile, when treated with OMDV^{VP-LB}, the expression of PDL1 increased by 30.2% (7.0% → 37.2%) and the expression of TIGIT increased by 10.7% (17.3% → 28.0%) (FIGs. 6E and 6F).

The splenocytes isolated from tumor-bearing mice were treated with bacterial extracellular vesicles and the expression of PDL1 and TIGIT was observed, and as a result, the expression of both PDL1 and TIGIT was significantly increased. Therefore, it can be reasonably expected that the anti-cancer effects may increase when bacterial extracellular vesicles are administered in combination with PDL1 and TIGIT inhibitors.

### INDUSTRIAL APPLICABILITY

According to the present invention, the composition including the bacterial extracellular vesicles and immune checkpoint inhibitors, either a single type or the combination of two or more types, as active ingredients exhibits a synergistic effect in cancer treatment compared to monotherapy using each agent. The composition also exhibits an effect of reducing adverse effects caused by high-dose administration of the single agents. As a result, the composition can be very usefully utilized for development of cancer therapeutic agents by providing the method for enhancing the cancer treatment effect, so that industrial applicability is very high.

## Claims

1. A pharmaceutical composition for preventing or treating cancer comprising a bacterial extracellular vesicle and an immune checkpoint inhibitor as active ingredients.

2. The pharmaceutical composition of claim 1, wherein the bacteria are Gram-negative bacteria or Gram-positive bacteria.

3. The pharmaceutical composition of claim 2, wherein the Gram-negative bacteria are selected from the group consisting of *Escherichia* genus, *Helicobacter* genus, *Hemophilus* genus, *Neisseria* genus, *Cyanobacterium* genus, *Klebsiella* genus, *Acetobacter* genus, *Acinetobacter* genus, *Enterobacter* genus, *Chlamydia* genus, *Vibrio* genus, *Pseudomonas* genus, *Salmonella* genus, *Thiobacter* genus, *Borrelia* genus, *Burkholderia* genus, *Serratia* genus, and *Treponema* genus.

4. The pharmaceutical composition of claim 2, wherein the Gram-positive bacteria are selected from the group consisting of *Bacillus* genus, *Nocardia* genus, *Clostridium* genus, *Propionibacterium* genus, *Actinomyces* genus, *Enterococcus* genus, *Corynebacterium* genus, *Listeria* genus, *Lactobacillus* genus, *Gardnerella* genus, *Mycobacterium* genus, *Mycoplasma* genus, *Staphylococcus* genus, *Streptomyces* genus, *Micrococcus* genus, *Streptococcus* genus.

5. The pharmaceutical composition of claim 1, wherein the bacteria are transformed bacteria.

6. The pharmaceutical composition of claim 5, wherein the bacteria are bacteria transformed to attenuate the toxicity of the extracellular vesicles.

7. The pharmaceutical composition of claim 5, wherein the bacteria are bacteria in which an endotoxin production gene is deleted or modified.

8. The pharmaceutical composition of claim 5, wherein the bacteria are bacteria transformed to target a specific cell or tissue.

9. The pharmaceutical composition of claim 1, wherein the bacteria are bacteria cultured in a chemically defined medium.

10. The pharmaceutical composition of claim 9, wherein the chemically defined medium is selected from the group consisting of M9 medium, Dulbecco's modified Eagle's medium (DMEM medium), and Roswell Park Memorial Institute medium 1640 (RPMI 1640 medium).

11. The pharmaceutical composition of claim 5, wherein the bacteria are bacteria transformed to express one or more selected from the group consisting of cell adhesion molecules, antibodies, targeting proteins, cell membrane fusion materials, and fusion proteins thereof.

12. The pharmaceutical composition of claim 11, wherein the cell membrane fusion material is human epidermal growth factor (EGF).

13. The pharmaceutical composition of claim 1, wherein the membrane of the bacterial extracellular vesicle further comprises components other than the cell membrane of the bacteria.

14. The pharmaceutical composition of claim 13, wherein the components other than the cell membrane of the bacteria are selected from the group consisting of targeting materials, cell membrane fusion materials, cyclodextrin, and polyethylene glycol.

15. The pharmaceutical composition of claim 1, wherein the membrane components of the bacterial extracellular vesicles are chemically modified.

16. The pharmaceutical composition of claim 15, wherein the membrane components of the bacterial extracellular vesicles are chemically modified using a thiol group or an amine group, or polyethyleneglycol chemically binds to the bacterial extracellular vesicles.

17. The pharmaceutical composition of claim 1, wherein the bacterial extracellular vesicle is isolated by a method selected from the group consisting of ultracentrifugation, density gradient ultracentrifugation, ultrafiltration, size-exclusion chromatography, ion exchange chromatography, immunoaffinity capture, microfluidics-based isolation, aqueous two-phase systems, and polymer-based precipitation.

18. The pharmaceutical composition of claim 1, wherein the immune checkpoint inhibitor is selected from the group consisting of antagonists of programmed cell death-1 (PD1), programmed cell death-ligand 1 (PDL1), programmed cell death-ligand 2 (PDL2), cluster of differentiation 27 (CD27), cluster of differentiation 28 (CD28), cluster of differentiation 70 (CD70), cluster of differentiation 80 (CD80), cluster of differentiation 86 (CD86), T cell immunoreceptor with Ig and ITIM domains (TIGIT), cluster of differentiation 137 (CD137), cluster of differentiation 276 (CD276), killer-cell immunoglobulin-like receptors (KIRs), lymphocyte-activation gene 3 (LAG3), tumor necrosis factor receptor superfamily, member 4 (TNFRSF4), glucocorticoid-induced TNFR-related protein (GITR), glucocorticoid-induced TNFR-related protein ligand (GITRL), 4-IBB ligand (4-1BBL), cytotoxic T lymphocyte associated antign-4 (CTLA4), adenosine A2A receptor (A2aR), V-set domain-containing T-cell activation inhibitor 1 (VTCN1), B- and T-lymphocyte attenuator (BTLA), indoleamine 2,3-dioxygenase (IDO), T-cell immunoglobulin domain and mucin-domain containing-3 (TIM3), V-domain Ig suppressor of T cell activation (VISTA), and killer cell lectin-like receptor subfamily A (KLRA), and combinations thereof.

19. The pharmaceutical composition of claim 18, wherein the immune checkpoint inhibitor is a protein, a peptide, an antibody, an antigen-binding fragment thereof, or an inhibitory nucleic acid.

20. The pharmaceutical composition of claim 19, wherein the inhibitory nucleic acid is siRNA, shRNA, or anti-sense RNA.

21. The pharmaceutical composition of claim 1, wherein the immune checkpoint inhibitor is selected from the group consisting of Durvalumab, Atezolizumab, Avelumab, Tremelimumab, Ipilimumab, Pembrolizumab, Nivolumab, Pidilizumab, BMS986016, Cemiplimab and Lirilumab,

22. The pharmaceutical composition of claim 1, wherein the bacterial extracellular vesicles and the immune checkpoint inhibitors are co-administered simultaneously, sequentially or separately.

23. The pharmaceutical composition of claim 1, wherein the cancer is selected from the group consisting of gastric cancer, lung cancer, non-small cell lung cancer, breast cancer, ovarian cancer, hepatic cancer, bronchial cancer, nasopharyngeal cancer, laryngeal cancer, pancreatic cancer, colorectal cancer, bladder cancer, colon cancer, cervical cancer, bone cancer, non-small cell bone cancer, hematologic malignancy, skin cancer, head or neck cancer, uterine cancer, rectal cancer, perianal cancer, colon cancer, fallopian tube cancer, endometrial cancer, vaginal cancer, cancer of the vulva, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, renal or ureteral cancer, renal cell carcinoma, renal pelvic cancer. salivary gland cancer, sarcoma, pseudomyxoma, hepatoblastoma, testicular cancer, glioblastoma, lip cancer, germ cell tumor of ovary, basal cell carcinoma, multiple myeloma, gallbladder cancer, choroidal melanoma, ampullar of vater cancer, peritoneal carcinoma, tongue cancer, small cell cancer, pediatric lymphoma, neuroblastoma, duodenal cancer, ureteral cancer, astrocytoma, meningioma, renal pelvis cancer, vulvar cancer, thymus cancer, central nervous system (CNS) tumor, primary central nervous system lymphoma, spinal cord tumor, brainstem glioma, and pituitary adenoma.

24. The pharmaceutical composition of claim 1, wherein the pharmaceutical composition is administered to mammals including humans, by an oral method or a parenteral method.

25. The pharmaceutical composition of claim 24, wherein the parenteral administration method is selected from the group consisting of intravenous, intramuscular, intraarterial, intramedullary, intrathecal, intracardiac, transdermal, subcutaneous, intraperitoneal, intranasal, intestinal, topical, sublingual, and rectal administration.

26. A use of a bacterial extracellular vesicle and an immune checkpoint inhibitor for preparing a cancer therapeutic agent.

27. A method for treating cancer comprising administering an effective amount of a composition comprising a bacterial extracellular vesicle and an immune checkpoint inhibitor as active ingredients to a subject in need thereof.
